# EUROPEAN PATENT APPLICATION

(11) **EP 3 449 818 A1**
(43) Date of publication of application: **06.03.2019**
(21) Application number: 17789383.1
(22) Date of filing: 19.04.2017
(51) Int. Cl.: A61B 5/02, A61B 5/022

(54) **DEVICE, TERMINAL AND BIOMETRIC INFORMATION SYSTEM**

(30) Priority: 27.04.2016 JP 2016089911
(71) Applicant: Asahi Kasei Kabushiki Kaisha, Chiyoda-ku Tokyo 101-8101 (JP)
(72) Inventor: OGAWA Shimpei, Tokyo 101-8101 (JP); NOGUCHI Yoshihiro, Tokyo 101-8101 (JP)
(74) Representative: Hoffmann Eitle
(86) International application number: PCT/JP2017/015775
(87) International publication number: WO 2017/188099

(57) **Abstract**

An apparatus that stores information on a pulse wave of a living body is provided, the apparatus including: a receiving unit that receives a bias signal and a fundamental wave signal of the pulse wave, respectively; and a storage unit that stores the bias signal and fundamental wave signal. A terminal is provided, the terminal including: an acquiring unit that acquires a pulse wave signal of a living body; a calculating unit that calculates a bias signal and a fundamental wave signal of the pulse wave from the pulse wave signal; and a transmitting unit that transmits the bias signal and the fundamental wave signal to an apparatus provided outside. In addition, a bioinformation system including an apparatus according to the present specification and a terminal according to the present specification is provided.

## Description

### BACKGROUND

### 1. TECHNICAL FIELD

The present invention relates to an apparatus, a terminal and a bioinformation system.

### 2. RELATED ART

It has been conventionally known to calculate and store the frequency, higher harmonic waves and the like of a fundamental wave in an apparatus for processing and storing pulse waves or sound waves (please see Patent Documents 1 to 4, for example).
Patent Document 1: Japanese Patent Application Publication No. H 7-148253
Patent Document 2: WO1999/026529
Patent Document 3: Japanese Patent Application Publication No. H 7-261798
Patent Document 4: Japanese Patent Application Publication No. 2015-118376

However, it is not possible, with conventional apparatuses, to efficiently compress and save data for reproducing original pulse waves because bias signals of pulse waves are not acquired.

### [General Disclosure]

A first aspect of the present invention provides an apparatus that stores information on a pulse wave of a living body, the apparatus including: a receiving unit that receives a bias signal and a fundamental wave signal of the pulse wave, respectively; and a storage unit that stores the bias signal and fundamental wave signal.

A second aspect of the present invention provides a terminal including: an acquiring unit that acquires a pulse wave signal of a living body; a calculating unit that calculates a bias signal and a fundamental wave signal of the pulse wave from the pulse wave signal; and a transmitting unit that transmits the bias signal and the fundamental wave signal to an apparatus provided outside.

A third aspect of the present invention provides a bioinformation system including: the processing apparatus according to the first aspect; and the terminal according to the second aspect.

The summary clause does not necessarily describe all necessary features of the embodiments of the present invention. The present invention may also be a sub-combination of the features described above.

### BRIEF DESCRIPTION OF THE DRAWINGS

FIG. 1 shows one example of the configuration of a bioinformation system 300 according to Example 1.
FIG. 2A shows one example of a pulse wave signal acquired from optical information of a living body.
FIG. 2B shows the amplitude and phase of a pulse wave signal after FFT.
FIG. 2C shows reproduced pulse wave signals of a living body.
FIG. 3 shows one example of the summary of the configuration of the bioinformation system 300 according to Example 2.
FIG. 4 shows the summary of the configuration of the bioinformation system 300 according to Example 3.
FIG. 5 shows one example of the configuration of the bioinformation system 300 according to Example 4.
FIG. 6 shows one example of the configuration of the bioinformation system 300 according to Example 5.
FIG. 7 shows one example of the configuration of the bioinformation system 300 according to Example 6.

### DESCRIPTION OF EXEMPLARY EMBODIMENTS

Hereinafter, (some) embodiment(s) of the present invention will be described. The embodiment(s) do(es) not limit the invention according to the claims, and all the combinations of the features described in the embodiment(s) are not necessarily essential to means provided by aspects of the invention.

### [Example 1]

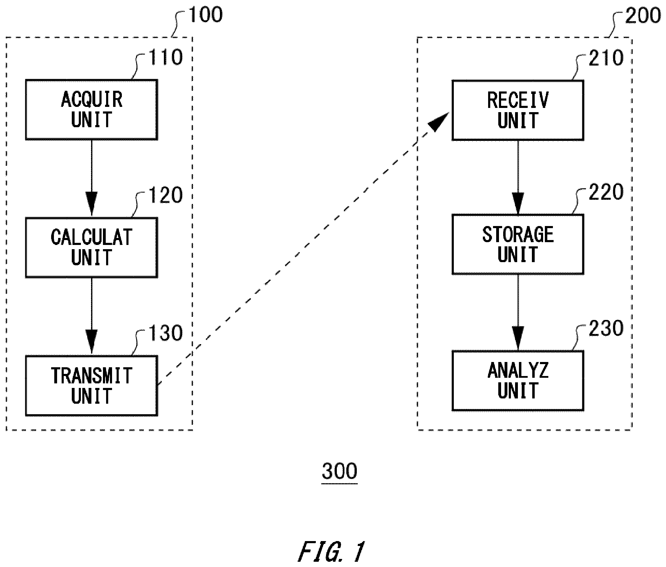

FIG. 1 shows one example of the configuration of a bioinformation system 300 according to Example 1. The bioinformation system 300 includes a terminal 100 and a processing apparatus 200.

The terminal 100 includes an acquiring unit 110, a calculating unit 120 and a transmitting unit 130. The terminal 100 is, but not limited to, a terminal such as a mobile phone, a smartphone or a wearable device. The terminal 100 may be a desktop personal computer or laptop personal computer.

The acquiring unit 110 acquires information on a living body. In one example, the acquiring unit 110 acquires optical information of a living body as information on a living body. The acquiring unit 110 may acquire optical information of an entire living body or may acquire optical information of a part of a living body. For example, the optical information is optical information on a living body acquired with a camera or optical element. The optical information may be image data such as a still image or moving image. In addition, the acquiring unit 110 acquires pulse wave information from optical information of a living body. The pulse wave information is information on a temporal waveform of pulsation of blood (that is, a pulse wave).

In one example, the acquiring unit 110 has a camera. The acquiring unit 110 photographs a living body with the camera to acquire optical information on the living body. For example, as pulse wave information of a living body, the acquiring unit 110 acquires a color-difference signal acquired from optical information of the living body. In addition, the acquiring unit 110 may acquire a luminance signal from optical information of a living body. If the acquiring unit 110 has a camera, in addition to pulse wave information, the acquiring unit 110 acquires image information from optical information. For example, as the image information, the acquiring unit 110 acquires the age, gender or the like of a living body estimated from an image of the living body. The optical information may be image data such as a still image or a moving image.

In addition, in another example, the acquiring unit 110 has an optical element consisting of a light-emitting unit and a light-receiving unit. The light-emitting unit and light-receiving unit are provided to a wearable device. The light-emitting unit has a light-emitting diode (LED) that irradiates a living body with light at a predetermined wavelength. In addition, the light-receiving unit has a photo diode (PD) that detects reflected light or transmitted light of light emitted by the LED. The acquiring unit 110 detects, with the PD, light corresponding to light emitted by the LED to acquire optical information of a living body. The LED radiates radiated light including light at a plurality of wavelengths. In one example, the LED radiates first wavelength light and second wavelength light in a time-division manner. The LED may radiate first wavelength light and second wavelength light alternately. In addition, the LED may radiate first wavelength light and second wavelength light simultaneously. For example, the LED radiates radiated light including light at the green and red wavelengths as first wavelength and second wavelength light.

The calculating unit 120 calculates a feature quantity of a living body from optical information acquired by the acquiring unit 110. The feature quantity of the living body is a feature related to a state of the living body which is expressed in a numeric form. For example, the feature quantity of the living body includes a feature quantity related to a pulse wave of the living body, a feature quantity related to a blood pressure of the living body, a feature quantity related to the age of the living body, a feature quantity related to motion of the living body and the like. In one example, the calculating unit 120 calculates a bias signal and a fundamental wave signal of a pulse wave by separating noise components from optical information of a living body. Time intervals of bias signals calculated by the calculating unit 120 are preferably 0.5 to 1.3 seconds. Time intervals of fundamental wave signals calculated by the calculating unit 120 are preferably 0.5 to 1.3 seconds. At this time, the time intervals of the bias signals and the time intervals of the fundamental wave signals may be different. For example, the calculating unit 120 is a semiconductor apparatus. The calculating unit 120 may be a large-scale integration (LSI).

In the present specification, a bias signal refers to a signal at a frequency of 0 Hz or a direct current component signal in pulse wave information. A bias signal indicates a blood volume of a living body. Thereby, the blood circulation state of the living body can be known. In addition, a fundamental wave signal refers to a signal at a frequency corresponding to the pulse rate of a living body in pulse wave information. The fundamental wave signal includes information on the pulse of a living body. The fundamental wave signal is, for example, the frequency of a fundamental wave, the amplitude of a fundamental wave or the phase of a fundamental wave. Pulse wave signals of a living body can be reproduced from bias signals and fundamental wave signals.

The transmitting unit 130 transmits, to the processing apparatus 200, a feature quantity of a living body calculated by the calculating unit 120. The transmitting unit 130 preferably transmits only necessary information in optical information of a living body acquired by the acquiring unit 110. Time intervals of bias signals transmitted by the transmitting unit 130 are preferably 0.5 to 1.3 seconds. Time intervals of fundamental wave signals transmitted by the transmitting unit 130 are preferably 0.5 to 1.3 seconds. At this time, the time intervals of the bias signals and the time intervals of the fundamental wave signals may be different. The transmitting unit 130 may transmit, to the processing apparatus 200, all the feature quantities of a living body calculated by the calculating unit 120 or may selectively transmit some of them to the processing apparatus 200. Thereby, the communication traffic volume between the terminal 100 and the processing apparatus 200 can be reduced. The transmitting unit 130 may collectively transmit bias signals and fundamental wave signals calculated in multiple calculations. In addition, the signals may be transmitted in an encrypted form or may be transmitted in association with terminal information. In one example, as a feature quantity of a living body, the transmitting unit 130 transmits bias signals and fundamental wave signals to the processing apparatus 200 provided outside. A transmission method used may be performed using wires or wirelessly, but is preferably a wireless transmission method in terms of portability. A wireless transmission method used may be, for example, WiFi, Bluetooth (registered trademark), infrared communication, ultrasonic communication or the like.

The processing apparatus 200 stores a feature quantity of a living body and analyzes information on the living body based on the feature quantity of the living body. The processing apparatus 200 includes a receiving unit 210, a storage unit 220 and an analyzing unit 230.

The receiving unit 210 receives a feature quantity of a living body acquired from optical information of a living body. The receiving unit 210 may receive information transmitted from a plurality of terminals. In addition, the receiving unit 210 may receive only information transmitted from a particular terminal.

The storage unit 220 stores a feature quantity of a living body received at the receiving unit 210. The storage unit 220 may selectively store feature quantities of a living body. In one example, the storage unit 220 selectively stores bias signals and fundamental wave signals of a pulse wave. Thereby, the storage unit 220 can compress the amount of data and store feature quantities of a living body efficiently. Time intervals of bias signals stored by the storage unit 220 are preferably 0.5 to 1.3 seconds. Time intervals of fundamental wave signals stored by the storage unit 220 are preferably 0.5 to 1.3 seconds. At this time, the time intervals of the bias signals and the time intervals of the fundamental wave signals may be different. In addition, the storage unit 220 stores the frequency of a fundamental wave signal. In this case, the storage unit 220 may store the frequency of a fundamental wave signal, and may not store the amplitude and phase of the fundamental wave signal. For example, the storage unit 220 is a cloud storage or server.

The analyzing unit 230 analyzes the state of a living body based on a feature quantity of the living body stored in the storage unit 220. In one example, the analyzing unit 230 analyzes the state of a living body based on information on pulse waves of a plurality of living bodies. For example, the analyzing unit 230 analyzes the state of a living body based on bias signals and fundamental wave signals stored in the storage unit 220. In addition, the analyzing unit 230 analyzes a feature quantity of a living body stored in the storage unit 220 to acquire the pulse rate, blood pressure, respiration or the like of the living body. For example, the analyzing unit 230 includes the artificial intelligence or machine learning function.

In addition, the analyzing unit 230 estimates the age of a living body from image information of a living body and corrects blood pressure information according to the age of the living body. Furthermore, the analyzing unit 230 may analyze pulse variation of a living body based on a signal. For example, the analyzing unit 230 calculates pulse variation of a living body based on fluctuation of bias signals or fundamental wave signals observed in a long time. Pulse variation of a living body is, for example, LFHF, HRV, Mayer waves or the like.

In one example, the storage unit 220 stores a higher harmonic wave signal of a pulse wave. A higher harmonic wave signal is a signal which is included in pulse wave information and is at a frequency higher than the frequency corresponding to the pulse rate of a living body. The storage unit 220 may store a higher harmonic wave signal at any frequency. In terms of data compression and utilization, the storage unit 220 preferably stores selectively a second higher harmonic wave signal and a third higher harmonic wave signal having frequencies twice or three times higher than the frequency corresponding to the pulse rate of a living body. In this case, the storage unit 220 may store the amplitude and phase of each higher harmonic wave signal. The phase of a higher harmonic wave signal may be a phase difference measured using the phase of a fundamental wave signal as a reference. In addition, the amplitude of a higher harmonic wave signal may be an amplitude ratio measured using the amplitude of a fundamental wave signal as a reference. Time intervals of higher harmonic wave signals stored by the storage unit 220 are preferably no shorter than 0.5 seconds. At this time, the time intervals of the bias signals and the time intervals of the fundamental wave signals may be different.

In one example, the storage unit 220 stores the reliability of a pulse wave. The reliability represents the quality of optical information acquired by the acquiring unit 110. For example, it may be the ratio between the sum of amplitudes of a fundamental wave and a higher harmonic wave and the average amplitude of the entire frequency band, and in such a case the smaller the noise is, the higher the reliability is.

In one example, the storage unit 220 stores a color-difference signal calculated from optical information of a living body. The storage unit 220 estimates the arterial oxygen saturation (SpO₂) of the living body based on the stored color-difference signal. The storage unit 220 stores a first color-difference signal acquired from image data of a living body, and a second color-difference signal different from the first color-difference signal. In addition, the storage unit 220 may store the sum of and difference between the first color-difference signal and the second color-difference signal, respectively. For example, the storage unit 220 stores color-difference signals Cb and Cr, respectively.

SpO₂ indicates the ratio between oxygenated hemoglobin HbO₂ and reduced hemoglobin Hb in blood of a living body. Oxygenated hemoglobin HbO₂ is hemoglobin bonded with oxygen, and reduced hemoglobin Hb is hemoglobin not bonded with oxygen. In other words, SpO₂ indicates the proportion of hemoglobin bonded with oxygen. Reduced hemoglobin Hb absorbs more light at the red wavelength than oxygenated hemoglobin HbO₂ does. SpO₂ is calculated utilizing this characteristic.

In one example, the storage unit 220 stores LFHF calculated from optical information of a living body. LFHF is a signal which is included in pulse information of a living body and has a frequency component lower than the frequency of a pulse rate. Here, LF indicates low frequency (LF), and HF indicates high frequency (HF). The processing apparatus 200 calculates the ratio between LF and HF to be able to judge the stress condition of a living body.

In one example, the storage unit 220 stores attribute information calculated from optical information of a living body. The attribute information is information indicating attributes of a living body, and includes the age and gender of the living body and information on personal identification. For example, the storage unit 220 stores attribute information acquired from a facial image of a living body. In addition, the storage unit 220 may store information based on the shape of a face acquired from a facial image of a living body in association with information on the pulse wave of the living body. Thereby, the processing apparatus 200 can identify what kind of a living body a stored feature quantity belongs to. In addition, the processing apparatus 200 can evaluate health of living bodies based on their feature quantities age by age.

In one example, the storage unit 220 stores acceleration information acquired from a living body at the time of measurement of a feature quantity of the living body. For example, the storage unit 220 stores acceleration information acquired from a living body at the time of measurement of bias signals and fundamental wave signals. Thereby, the processing apparatus 200 can precisely judge a motion state or sleep state using the acceleration information.

In one example, the storage unit 220 controls the processing frame length of a pulse wave and stores a feature quantity of a living body. The calculating unit 120 controls the frame length set when processing is performed by fast Fourier transform (FFT). For example, the calculating unit 120 makes the frame length long if pulse waves are relatively stable during a period of sleep or the like and it is desired to record variation observed in a long time. Then, the storage unit 220 may store a start clock time indicating the starting point of the processing frame of a pulse wave and an end clock time indicating the clock time of the end point of the processing frame.

In this manner, the processing apparatus 200 of the present example selectively stores any feature quantity among feature quantities of a living body such as a bias signal of a pulse wave, a pulse rate, the amplitude and phase of a fundamental wave signal, the amplitude and phase of a higher harmonic wave signal or reliability. In addition, the processing apparatus 200 may store clock time information, LFHF, blood pressure information and acceleration information. The processing apparatus 200 of the present example selectively stores a feature quantity of a living body according to information necessary for a user so that the data communication traffic volume and capacity at the time of storage can be reduced.

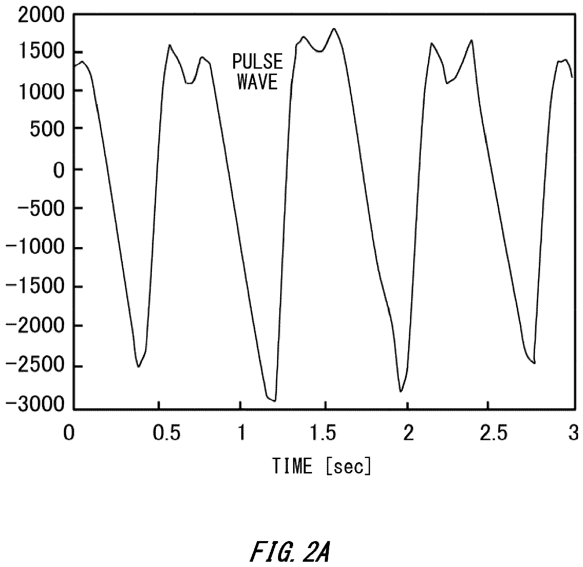

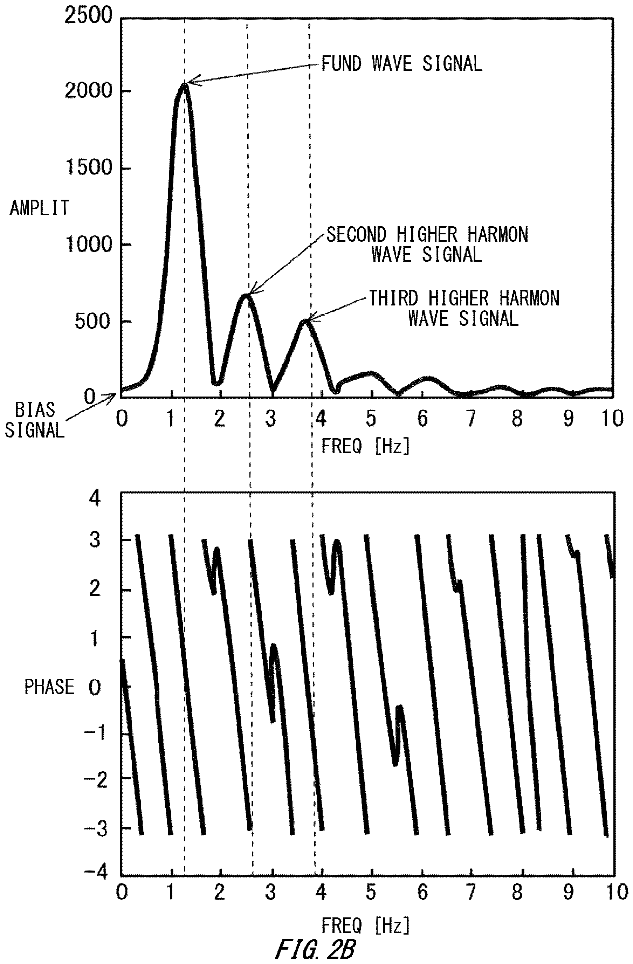

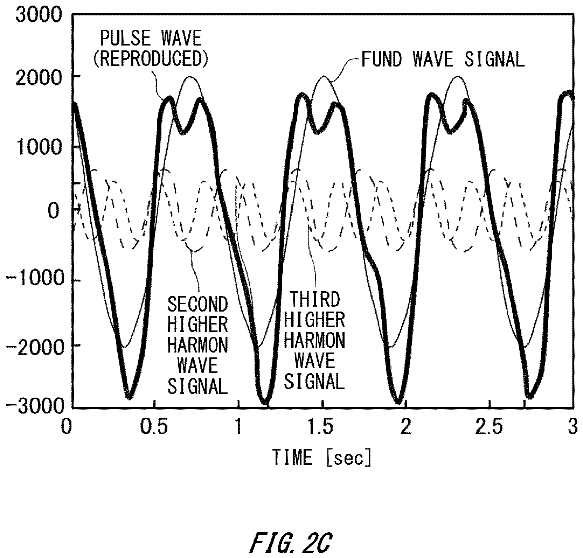

FIG. 2A shows one example of a pulse wave signal acquired from optical information of a living body. The vertical axis indicates the strength of the pulse wave signal, and the horizontal axis indicates time [sec]. FIG. 2B shows the amplitude and phase of a pulse wave signal after FFT. The vertical axes indicate the amplitude and phase, and the horizontal axes indicate the frequency [Hz]. FIG. 2C shows reproduced pulse wave signals of a living body. The vertical axis indicates the strength of the pulse waves, and the horizontal axis indicates time [sec]. The respective curves indicate a pulse wave, a fundamental wave signal, a second higher harmonic wave signal and a third higher harmonic wave signal of the living body.

The pulse rate of a living body is calculated based on color-difference signals. In one example, the calculating unit 120 performs FFT processing on a color-difference signal Cb+Cr to calculate the pulse rate of the living body. The calculating unit 120 of the present example calculates, as the pulse rate, the frequency at which the amplitude becomes largest in the frequency band of 0.5 Hz to 3.3 Hz inclusive.

The fundamental wave signal is a signal at the frequency corresponding to the pulse rate. The calculating unit 120 calculates the amplitude and phase of a frequency component equivalent to the pulse rate, as the amplitude and phase of the fundamental wave signal, respectively. In one example, the calculating unit 120 calculates the amplitude and phase of the fundamental wave signal based on color-difference signals Cb+Cr and Cb-Cr. The amplitude of the fundamental wave signal is correlated with a cardiac output. In addition, the phase of the fundamental wave signal is used for reproduction of continuous pulse waves.

The second higher harmonic wave signal is a signal having a frequency component which is twice the frequency component of the pulse rate. The calculating unit 120 calculates the amplitude and phase of the second higher harmonic wave signal based on the color-difference signals Cb+Cr and Cb-Cr. That is, the calculating unit 120 calculates the amplitude and phase of a frequency component equivalent to the rate which is twice the pulse rate, as the amplitude and phase of the second higher harmonic wave signal, respectively.

The third higher harmonic wave signal is a signal having a frequency component which is three times the frequency component of the pulse rate. The calculating unit 120 calculates the amplitude and phase of the third higher harmonic wave signal based on the color-difference signals Cb+Cr and Cb-Cr. That is, the calculating unit 120 calculates the amplitude and phase of a frequency component which is three times the frequency component of the pulse rate, as the amplitude and phase of the third higher harmonic wave signal, respectively.

For example, the bioinformation system 300 uses, in combination, the second higher harmonic wave signal and third higher harmonic wave signal in addition to the fundamental wave signal to be able to calculate an acceleration pulse wave. That is, the bioinformation system 300 acquires higher harmonic wave signals to be able to acquire information on the shape of pulse waves. In addition, the higher harmonic wave signals are correlated with vascular ages. Because of this, the bioinformation system 300 acquires the higher harmonic wave signals to be able to estimate vascular ages.

### [Example 2]

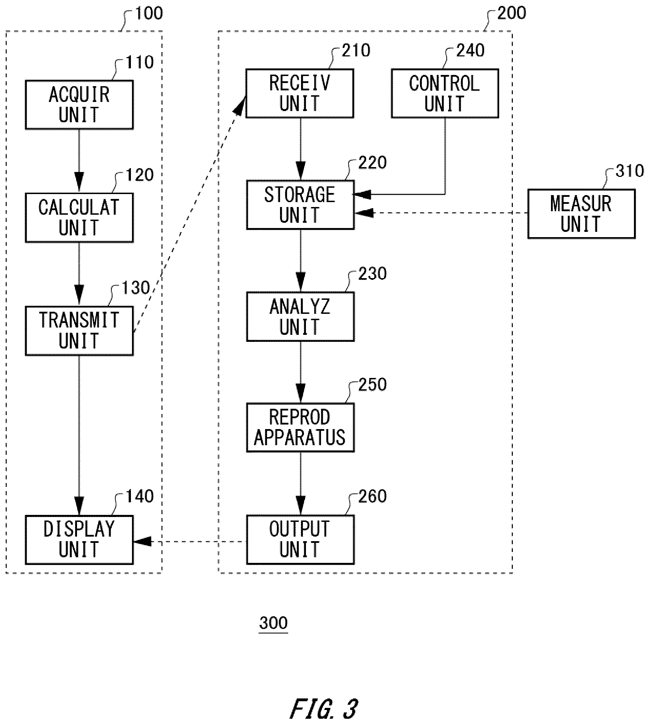

FIG. 3 shows one example of the summary of the configuration of the bioinformation system 300 according to Example 2. The bioinformation system 300 of the present example includes the terminal 100, the processing apparatus 200 and a measuring unit 310. The terminal 100 includes the acquiring unit 110, the calculating unit 120, the transmitting unit 130 and a display unit 140. In addition, the processing apparatus 200 includes the receiving unit 210, the storage unit 220, the analyzing unit 230, a control unit 240 and an output unit 260.

The display unit 140 displays any information to a user. Any information is input to the display unit 140 from the transmitting unit 130 and output unit 260. In one example, a feature quantity of a living body is input to the display unit 140 from the transmitting unit 130. The transmitting unit 130 may input, to the display unit 140, information which is the same as a feature quantity of a living body to be transmitted to the processing apparatus 200 or may input different information to the display unit 140. The display unit 140 displays, to a user, information input from the transmitting unit 130 or processing apparatus 200.

The control unit 240 controls the frequencies of storing, in the storage unit 220, feature quantities of a living body transmitted from the transmitting unit 130. In one example, the control unit 240 makes storage frequencies of the storage unit 220 different for different feature quantities of a living body. Thereby, the control unit 240 prevents unnecessary feature quantities of a living body from being input to the processing apparatus 200, and reduces wasteful processes of the processing apparatus 200 to reduce consumed current. For example, when large noise is included in pulse waves of a living body, during a period of sleep of a living body or when the remaining capacity of a battery of the terminal 100 is small, the control unit 240 reduces the process frequency.

In one example, the control unit 240 controls the storage frequency for each feature quantity of a living body according to temporal change amounts of feature quantities of the living body. For example, if temporal change amounts of a feature quantity of a living body are large, the control unit 240 increases the frequency of storing the feature quantity of the living body, and if temporal change amounts of a feature quantity of a living body are small, the control unit 240 reduces the frequency of storing the feature quantity of the living body. In one example, because temporal change amounts of the blood volume of a living body are larger than temporal change amounts of a pulse waves, the control unit 240 performs control such that the frequency of storing bias signals becomes higher than the frequency of storing fundamental wave signals and higher harmonic wave signals. For example, the control unit 240 causes the storage unit 220 to store bias signals every second, and causes the storage unit 220 to store fundamental wave signals and higher harmonic wave signals every minute. Thereby, the processing apparatus 200 can reduce the amount of data to be saved in the storage unit 220.

In addition, in one example, the control unit 240 controls the storage frequency of the storage unit 220 according to acceleration information of a living body. If temporal changes in acceleration information are large, the control unit 240 increases the frequency of storage of bias signals and fundamental wave signals by the storage unit 220. Thereby, information of a living body when motion of the living body is intense can be measured in more detail.

Although in the present example, the control unit 240 is included in the processing apparatus 200, the terminal 100 may include a control unit. In such a case, the calculating unit 120 may control the frequency of calculating feature quantities of a living body, and the transmitting unit 130 may control the frequency of transmitting feature quantities of a living body. Thereby, the computation amount and communication traffic volume can be reduced.

A reproducing apparatus 250 reproduces a pulse wave of a living body based on a feature quantity of the living body stored in the storage unit 220. The reproducing apparatus 250 reproduces a pulse wave of a living body based on a feature quantity of the living body stored in the storage unit 220. In one example, the reproducing apparatus 250 reproduces a pulse wave of a living body by inverse Fourier transform on a bias signal and a fundamental wave signal stored in the storage unit 220. In addition, the reproducing apparatus 250 may reproduce a pulse wave of a user according to a request from a user. For example, the reproducing apparatus 250 reproduces a pulse wave at a clock time corresponding to a request of a user. The reproducing apparatus 250 outputs a signal of the reproduced pulse wave to the output unit 260.

The output unit 260 transmits information that the processing apparatus 200 has to the terminal 100 of a user. The output unit 260 outputs a pulse wave of a living body reproduced by the reproducing apparatus 250 to the terminal 100. The output unit 260 may output, to the terminal 100, information of a living body calculated by the analyzing unit 230 by analysis. In addition, the output unit 260 may output, to the terminal 100, a feature quantity of a living body stored in the storage unit 220. In one example, according to a request from the terminal 100, the output unit 260 outputs information specified by the terminal 100. For example, the output unit 260 outputs, to the terminal 100, any of a pulse rate, a systolic blood pressure, a diastolic blood pressure and a combination of them. In addition, the output unit 260 may output, to the terminal 100, any of a motion state, a health condition, SpO₂, and overall health evaluation of a living body or a combination of them. The output unit 260 may output a result of comparison between a past state of a living body and a current state of the living body.

The measuring unit 310 measures various reference values of a living body, while at the same time measuring a pulse wave. The reference values of a living body indicate the state of a living body. The reference values of a living body may be information other than a pulse wave of a living body. In one example, as the reference values, the measuring unit 310 measures any of a motion amount, an activity amount, resistance of skin or biological impedance of a living body or a combination of them. The bioinformation system 300 has the measuring unit 310, and so new information formed by combining a feature quantity of a living body and a reference value can be provided. Although the measuring unit 310 of the present example is provided independently of the terminal 100, it may be provided inside the terminal 100.

For example, the measuring unit 310 acquires a facial age which is an estimate of the age of a living body based on a luminance signal Y of a facial video acquired with a camera. In addition, the measuring unit 310 may estimate the gender, body temperature, emotion or gaze based on a facial video photographed with a camera. If the acquiring unit 110 has a camera, the measuring unit 310 may be used in combination with the camera of the acquiring unit 110.

In one example, the measuring unit 310 measures blood pressure information of a living body. In this case, the measuring unit 310 has an upper arm cuff-type sphygmomanometer. In the present specification, the blood pressure information is a cuff blood pressure of a living body measured using a cuff. The blood pressure information of a living body may include a systolic blood pressure and a diastolic blood pressure. In addition, the measuring unit 310 may acquire clock time information about the time at which a blood pressure of a living body is measured. The measuring unit 310 measures a blood pressure of a wrist, leg or a fingertip. The measuring unit 310 may measures any of a body weight, a body fat percentage, SpO₂ or a body temperature. The storage unit 220 of the present example stores blood pressure information of a living body at the time of measurement of a bias signal and a fundamental wave signal. The storage unit 220 stores blood pressure information of a living body to be able to correct and update a formula for estimating consecutive blood pressures based on a pulse wave. In addition, by using blood pressure information and a pulse wave of a living body in combination, the degree of arteriosclerosis can be estimated highly precisely.

### [Example 3]

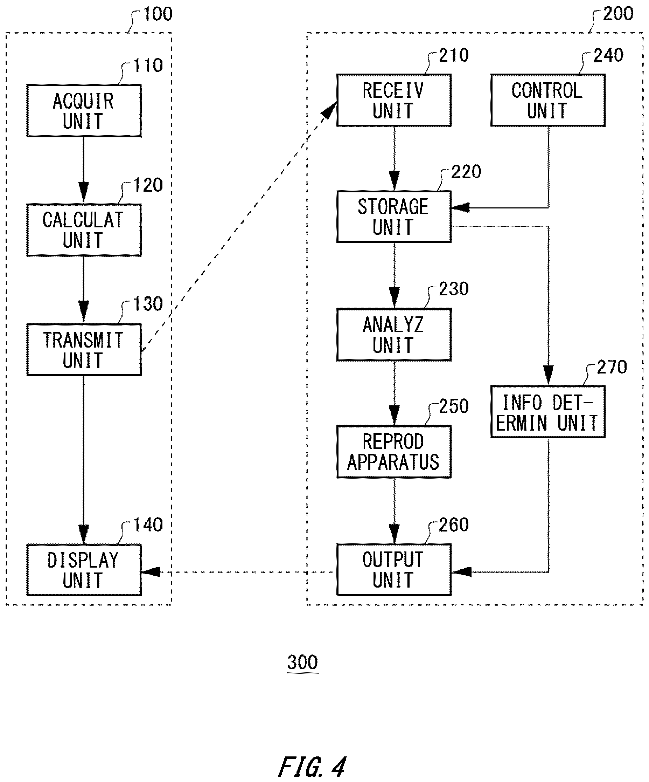

FIG. 4 shows the summary of the configuration of the bioinformation system 300 according to Example 3. The processing apparatus 200 of the present example further includes an information determining unit 270, in addition to the configuration of the processing apparatus 200 according to Example 2.

Based on a feature quantity of a living body stored in the storage unit 220, the information determining unit 270 determines information to be transmitted from the processing apparatus 200 to the terminal 100. In one example, if a bias signal or fundamental wave signal stored in the storage unit 220 shows an abnormal value, the information determining unit 270 causes the output unit 260 to output a feature quantity of a living body related to the abnormal value. Thereby, the terminal 100 can display information on the abnormal value to a user.

In addition, the information determining unit 270 causes the display unit 140 to display an advertisement based on a feature quantity of a living body stored in the storage unit 220. In this case, the information determining unit 270 is caused to display an advertisement corresponding to a health condition of a living body known through analysis by the analyzing unit 230. For example, if the stress condition of a living body is not good, the information determining unit 270 causes the terminal 100 to display an advertisement of a product related to stress. Other than advertisements, contents such as videos or music, for example, may be displayed.

Furthermore, the information determining unit 270 may learn optimal information according to a feature quantity of a living body stored in the storage unit 220, and optimize information to be output from the output unit 260. That is, the information determining unit 270 determines information to be output from the output unit 260 in view also of a feature quantity of a living body other than a user of the terminal 100 stored in the storage unit 220. Thereby, the information determining unit 270 can cause the output unit 260 to output information optimal for a user of the terminal 100. For example, the information determining unit 270 causes the output unit 260 to output a result of comparison with feature quantities of other users.

### [Example 4]

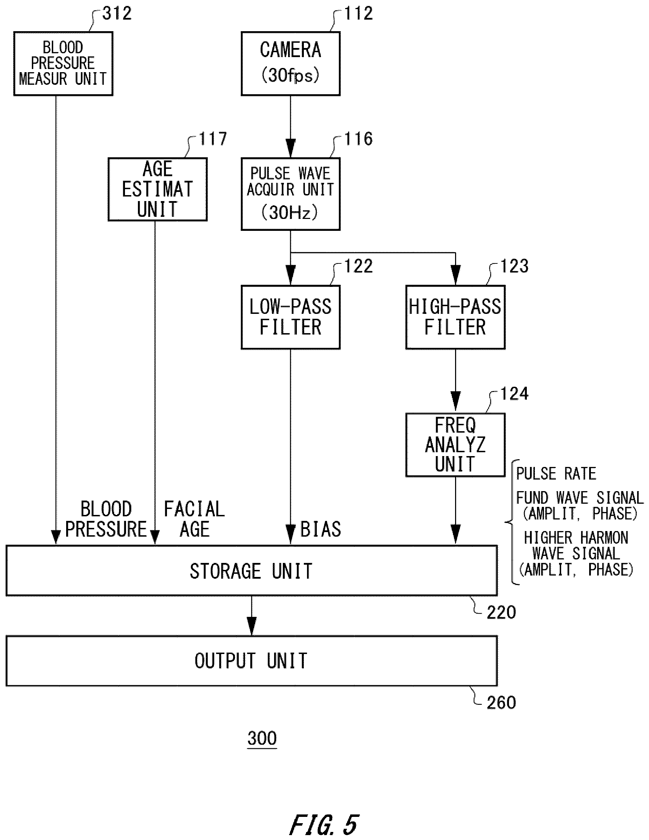

FIG. 5 shows one example of the configuration of the bioinformation system 300 according to Example 4. The bioinformation system 300 of the present example includes a camera 112, a pulse wave acquiring unit 116, an age estimating unit 117, a low-pass filter 122, a high-pass filter 123, a frequency analyzing unit 124, the storage unit 220, the output unit 260 and a blood pressure measuring unit 312.

The camera 112 photographs a living body. The camera 112 may have a CMOS or a CCD. In one example, the camera 112 photographs the face of a living body. The camera 112 may photograph the nose, fingers, arms or legs of a living body or multiple portions among them. Because the camera 112 photographs a video of a living body in a contactless manner, the burden of measurement on a living body can be reduced. In addition, the camera 112 can acquire information of a plurality of wavelengths at once. A video of a living body photographed by the camera 112 allows personal identification or an estimation of the age and gender of a living body.

In addition, the camera 112 photographs a living body at a predetermined frame rate according to a feature quantity of the living body calculated by the terminal 100. The camera 112 of the present example photographs a living body at the frame rate of 30 fps. In addition, the frame rate of the camera 112 may be 60 fps or 120 fps. However, the frame rate of the camera 112 is preferably no lower than 5 fps in order to calculate a fundamental wave signal and higher harmonic wave signals of a pulse wave.

The pulse wave acquiring unit 116 acquires information on a pulse wave from an image of a living body photographed by the camera 112. The pulse wave acquiring unit 116 acquires a color-difference signal through color conversion based on an image photographed by the camera 112. In addition, the pulse wave acquiring unit 116 may acquire a luminance signal.

For example, the pulse wave acquiring unit 116 acquires a luminance signal Y to be able to locate the position of a face through highly precise facial recognition. In addition, the pulse wave acquiring unit 116 may locate the position of the face of a living body from an image photographed by the camera 112. Then, the pulse wave acquiring unit 116 acquires a color-difference signal Cb+Cr to be able to accurately calculate a pulse rate. The pulse wave acquiring unit 116 acquires a color-difference signal Cb-Cr to be able to estimate SpO₂ by using it in combination with Cb+Cr.

The pulse wave acquiring unit 116 acquires the sum total of color-difference signals Cb+Cr weighted in a predetermined range (that is, a video pulse wave) and a clock time of photographing. In addition, the pulse wave acquiring unit 116 acquires the sum total of color-difference signals Cb-Cr weighted in a predetermined range and a clock time of photographing. Thereby, the pulse wave acquiring unit 116 acquires color-difference signals associated with clock times of photographing.

Furthermore, the pulse wave acquiring unit 116 re-samples signals input from the camera 112. The pulse wave acquiring unit 116 sets a video pulse wave to 30 Hz based on a clock time of photographing. Thereby, the pulse wave acquiring unit 116 can correct fluctuation of intervals between clock times of photographing.

The pulse wave acquiring unit 116 samples signals of a living body at a sampling frequency of 30 Hz. In addition, the pulse wave acquiring unit 116 may sample signals of a living body at a sampling frequency of 60 Hz. The pulse wave acquiring unit 116 performs sampling at a sampling frequency of 20 to 100 Hz to be able to detect higher harmonic waves and additionally to reduce the load of latter computation.

In addition, the pulse wave acquiring unit 116 performs sampling with a pulse wave frame length of 4.3 seconds (= 128 samples). By setting the pulse wave frame length to be no shorter than 2.0 seconds, the pulse wave acquiring unit 116 can enhance reliability. Furthermore, the pulse wave acquiring unit 116 performs sampling with a pulse wave frame movement width of 1.0 second (= 30 samples). By setting the pulse wave frame movement width to 0.5 to 1.3 seconds, the pulse wave acquiring unit 116 can acquire low frequency components from feature quantity variation and additionally reduce the load of computation.

About a bias signal, the pulse wave acquiring unit 116 acquires, as a start clock time or an end clock time respectively, a clock time of the starting point or end point of a processing frame. In addition, about a bias signal of a pulse wave, the pulse wave acquiring unit 116 acquires, as a bias signal, a video pulse wave after a low-pass process on the start clock time is performed, for each of Cb+Cr and Cb-Cr. The bias signal is correlated with a blood volume.

The calculating unit 120 includes the low-pass filter 122, the high-pass filter 123 and the frequency analyzing unit 124. The calculating unit 120 may perform zero-padding FFT with a small amount of samples.

The low-pass filter 122 takes out only low-frequency components from an input signal. In one example, the low-pass filter 122 takes out signals in a low-frequency domain at a cutoff frequency of 0.6 Hz. Thereby, accurate calculation of a bias signal becomes possible.

The high-pass filter 123 takes out only high-frequency components from an input signal. In one example, the high-pass filter 123 takes out signals in a high-frequency domain at a cutoff frequency of 0.6 Hz. Thereby, accurate calculation of a pulse rate becomes possible.

The frequency analyzing unit 124 perform FFT processing. The frequency analyzing unit 124 standardizes color-difference signals Cb+Cr and color-difference signals Cb-Cr. Thereby, the frequency analyzing unit 124 subtracts a certain value from a high-pass video pulse wave in a processing frame to make the average zero. Next, the frequency analyzing unit 124 performs window function processing. In one example, the high-pass filter 123 performs multiplication with a Hunning window function having a processing frame length. By zero-padding, the frequency analyzing unit 124 adds data of zero before or after a processing frame until the number of samples becomes 1024. The frequency analyzing unit 124 executes FFT at points, the number of which is a power of two. In one example, the frequency analyzing unit 124 executes FFT at 1024 points.

Because the bioinformation system 300 of the present example has the camera 112, it can acquire pulse wave information of a living body in a contactless manner and in a short time. In addition, because the bioinformation system 300 of the present example acquires a video of a living body with the camera 112, it can perform facial recognition and age estimation simultaneously.

The blood pressure measuring unit 312 measures the blood pressure of a living body. The blood pressure measuring unit 312 is one example of the measuring unit 310 to measure a reference value of a living body. The blood pressure measuring unit 312 acquires blood pressure information of a living body at the time of acquisition of a feature quantity of the living body with the camera 112. For example, the blood pressure measuring unit 312 acquires blood pressure information of a living body at the time of measurement of a bias signal and a fundamental wave signal. The blood pressure measuring unit 312 measures any of a clock time at which a feature quantity of a living body is measured, a pulse rate, a systolic blood pressure or a diastolic blood pressure or a combination of them.

The analyzing unit 230 calculates respiration and the stress condition based on a bias signal or pulse rate. The analyzing unit 230 calculates a vascular age based on a facial age and the amplitude and phase of higher harmonic wave signals. The analyzing unit 230 judges a general health condition based on these analysis results and a blood pressure (cuff-type). In addition, because the analyzing unit 230 is provided in the processing apparatus 200, it can perform comparison with other measured people who are about the same facial age.

The storage unit 220 acquires a photographing start clock time or end clock time at the same time when it acquires optical information of a living body. In addition, the storage unit 220 stores an estimated age of a living body input from the age estimating unit 117. In one example, the storage unit 220 stores a facial age of a living body. In addition, the storage unit 220 stores a bias signal input from the low-pass filter 122. The bias signal is calculated from color-difference signals Cb+Cr and Cb-Cr. The storage unit 220 stores a pulse rate, a fundamental wave signal and higher harmonic wave signals from the frequency analyzing unit 124. The storage unit 220 stores the amplitude and phase of the fundamental wave signal. In addition, the storage unit 220 stores the amplitude and phase of the higher harmonic wave signals. In one example, the fundamental wave signal and higher harmonic wave signals are calculated from the color-difference signals Cb+Cr and Cb-Cr.

The output unit 260 transmits a reproduced pulse wave to the terminal 100 of a user. The output unit 260 outputs, to the terminal 100, information measured by the terminal 100, for example, a pulse rate, a systolic blood pressure, a diastolic blood pressure or the like. In addition, the output unit 260 may output information analyzed and calculated by the analyzing unit 230, for example, respiration, a stress condition, a vascular age, overall health evaluation or the like.

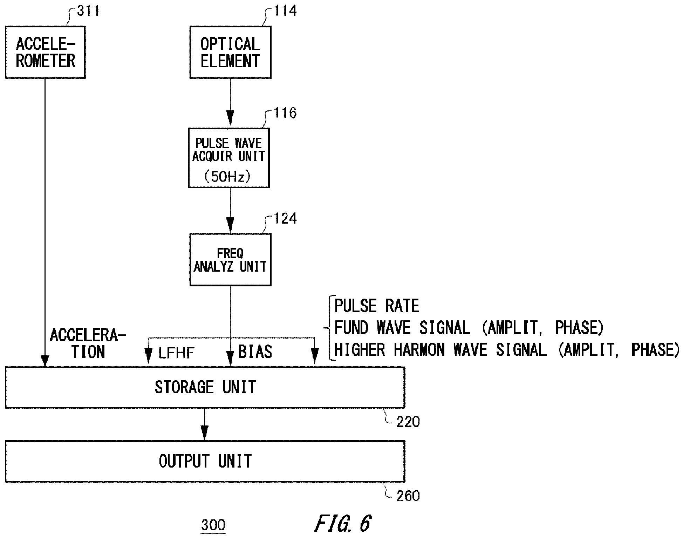

[Example 5] FIG. 6 shows one example of the configuration of the bioinformation system 300 according to Example 5. The bioinformation system 300 of the present example includes an optical element 114, the pulse wave acquiring unit 116, the frequency analyzing unit 124, the storage unit 220, the output unit 260 and an accelerometer 311.

The optical element 114 is one example of the acquiring unit 110. The optical element 114 includes a photoplethysmography (PPG) sensor having a light-emitting unit and a light-receiving unit. The light-emitting unit radiates radiated light including light at a plurality of wavelengths. In one example, the light-emitting unit radiates first wavelength light and second wavelength light in a time-division manner. The light-emitting unit may radiate first wavelength light and second wavelength light alternately. In addition, the light-emitting unit may radiate first wavelength light and second wavelength light simultaneously. The light-emitting unit of the present example has an LED to radiate radiated light at the green and red wavelengths. In addition, the light-emitting unit may emit light at the green wavelength, red wavelength or infrared wavelength or wavelengths which are a combination of them. The light-receiving unit has a photo diode. In addition, the light-receiving unit may have a phototransistor. The optical element 114 acquires a pulse wave at a wrist of a living body. In addition, the optical element 114 may acquire a pulse wave at fingers or legs or at a plurality of portions among them. The optical element 114 of the present example is mounted on a wearable device to be able to be worn always, so it is effective for measurement over a long time.

The accelerometer 311 acquires acceleration information of a living body. The bioinformation system 300 can simultaneously measure acceleration information and pulse wave information of a living body. In addition, the bioinformation system 300 measures the acceleration information and pulse wave information of a living body in association with each other. The transmitting unit 130 transmits, to the processing apparatus 200, a feature quantity of a living body acquired by the acquiring unit 110 and acceleration information of the living body in association with each other. In one example, the transmitting unit 130 transmits the bias signal and the fundamental wave signal in association with the acceleration information.

Because the optical element 114 radiates light at the stable green wavelength, it can accurately calculate the pulse rate of a living body. In addition, SpO₂ is estimated by the optical element 114 using light in the red wavelength range and light in the green wavelength range in combination. In this case, the optical element 114 sets the sampling frequency to 20 to 100 Hz. Thereby, the optical element 114 is able to detect higher harmonic wave signals and additionally to reduce the load of latter computation. The optical element 114 of the present example acquires optical information of a living body at the sampling frequency of 50 Hz.

The pulse wave acquiring unit 116 performs sampling of signals of a living body at the sampling frequency of 50 Hz. The pulse wave acquiring unit 116 performs sampling at the sampling frequency of 20 to 100 Hz to be able to detect higher harmonic waves and to reduce the load of latter computation.

In addition, the pulse wave acquiring unit 116 performs sampling with a pulse wave frame length of 41.0 second (= 2048 samples). In one example, the pulse wave acquiring unit 116 calculates LFHF with a pulse wave frame length which is set to be no shorter than 25 seconds. Furthermore, the pulse wave acquiring unit 116 performs sampling with a pulse wave frame movement width of 1.0 second (= 50 samples). By setting the pulse wave frame movement width to be no longer than 1.3 seconds, the pulse wave acquiring unit 116 acquires low frequency components from feature quantity variation. About a bias signal, the pulse wave acquiring unit 116 of the present example acquires, as a start clock time and an end clock time respectively, a clock time of the starting point and end point of a processing frame.

The frequency analyzing unit 124 executes FFT processing. Thereby, the frequency analyzing unit 124 calculates a pulse rate, a fundamental wave signal, higher harmonic wave signals, a bias signal and LFHF. The frequency analyzing unit 124 of the present example performs FFT processing on a signal in the green wavelength range and a signal in the red wavelength range. For example, the frequency analyzing unit 124 performs FFT on a video pulse wave of a processing frame at 2048 points.

The frequency analyzing unit 124 outputs LFHF to the storage unit 220. LFHF is calculated by the frequency analyzing unit 124 based on low frequency components in the green wavelength range and red wavelength range. Based on FFT processing, the frequency analyzing unit 124 calculates an amplitude equivalent to the frequency of 0.05 to 0.15 Hz as LF and an amplitude equivalent to the frequency of 0.15 to 0.40 Hz as HF. LFHF is correlated with stresses of a living body. Accordingly, the bioinformation system 300 calculates LFHF to be able to estimate the stress condition of a living body.

The frequency analyzing unit 124 outputs a bias signal to the storage unit 220. The bias signal is calculated using FFT based on the amplitude of a frequency equivalent to 0 Hz.

The frequency analyzing unit 124 outputs, to the storage unit 220, a pulse rate, a fundamental wave signal and higher harmonic wave signals. The pulse rate is calculated by the frequency analyzing unit 124 based on a signal in the green wavelength range. The frequency analyzing unit 124 acquires, as a pulse rate, a frequency at which the amplitude becomes the largest from within a range of 0.5 Hz to 3.3 Hz inclusive by FFT on a signal in the green wavelength range.

The fundamental wave signal and higher harmonic wave signals are calculated in a similar manner to that in the case of Example 1. In the present example, the amplitudes and phases of the higher harmonic wave signals are calculated by detecting light at the green and red wavelengths. The phases of the higher harmonic wave signals may be phase differences measured using the phase of the fundamental wave signal as a reference. In addition, the amplitudes of the higher harmonic wave signals may be amplitude ratios measured using the amplitude of the fundamental wave signal as a reference.

The acceleration is acquired by the accelerometer 311. The accelerometer 311 may acquire tri-axial acceleration (x, y, z). The accelerometer 311 outputs the acquired acceleration to the storage unit 220.

The storage unit 220 stores a start clock time or end clock time of processed data. In addition, the storage unit 220 stores: a bias signal acquired by the optical element 114, the pulse rate, the amplitude and phase of the fundamental wave signal, the amplitudes and phases of the higher harmonic wave signals; and LF and HF. In addition, the storage unit 220 may store acceleration information of a living body acquired with the accelerometer 311.

The analyzing unit 230 analyzes a motion state based on a pulse rate and tri-axial acceleration. In addition, the analyzing unit 230 analyzes a sleep state of a living body based on LFHF and tri-axial acceleration. The analyzing unit 230 calculates SpO₂ based on the amplitude of a fundamental wave signal. The analyzing unit 230 may perform overall health evaluation based on these analysis results.

The output unit 260 transmits a reproduced pulse wave to the terminal 100 of a user. The output unit 260 may output, to the terminal 100, information measured by the terminal 100, for example, any of a motion state, a health condition, SpO₂, and overall health evaluation of a living body or a combination of them.

Because the bioinformation system 300 of the present example is a wearable type system having an LED and a PD, it can acquire pulse wave information of a living body in contact with the living body and over a long time. In addition, the bioinformation system 300 is able to be used in combination with the accelerometer 311, and various data utilization in combination with acceleration information of a living body becomes possible.

The bioinformation system 300 according to the present specification can utilize information stored in the storage unit 220 for various uses. For example, the bioinformation system 300 can utilize information of a pulse wave and blood pressure stored in the storage unit 220 for an algorithm that derives a blood pressure estimation method based on the pulse wave.

In addition, the bioinformation system 300 can utilize information of a pulse wave and acceleration stored in the storage unit 220 for antinoise measures to reduce noise caused by body movement. Furthermore, the bioinformation system 300 can be utilized for bioinformation calculation in which a waveform of a pulse wave in a time domain is reproduced and displayed, and bioinformation is calculated based on a feature quantity or the waveform.

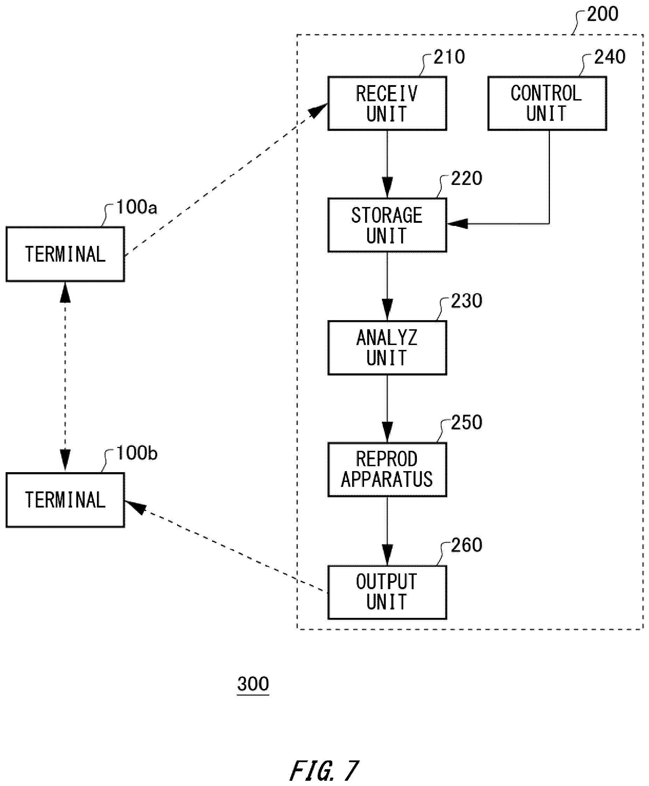

[Example 6] FIG. 7 shows one example of the configuration of the bioinformation system 300 according to Example 6. The bioinformation system 300 of the present example includes two terminals, a terminal 100a and a terminal 100b.

The terminal 100a detects a pulse wave of a living body. The terminal 100b is registered as a terminal corresponding to the terminal 100a. In one example, the terminal 100a calculates a feature quantity of a child, and the terminal 100b is carried by his/her parent.

The control unit 240 controls the storage frequency of the storage unit 220 according to the distance between the terminal 100a and the terminal 100b. For example, if the distance between the terminal 100a and the terminal 100b is sufficiently short, the bioinformation system 300 stops storage of feature quantities of a living body of the terminal 100a. That the distance between the terminal 100a and the terminal 100b is sufficiently short means that the distance is short enough for a parent to be able to monitor his/her child. On the other hand, if the terminal 100a is kept away from the terminal 100b, the bioinformation system 300 outputs, from the processing apparatus 200 to the terminal 100b, a feature quantity of a living body calculated by the terminal 100b. Thereby, a parent carrying the terminal 100b can remotely monitor a feature quantity of his/her child.

While the embodiments of the present invention have been described, the technical scope of the invention is not limited to the above described embodiments. It is apparent to persons skilled in the art that various alterations and improvements can be added to the above-described embodiments. It is also apparent from the scope of the claims that the embodiments added with such alterations or improvements can be included in the technical scope of the invention.

The operations, procedures, steps, and stages of each process performed by an apparatus, system, program, and method shown in the claims, embodiments, or diagrams can be performed in any order as long as the order is not indicated by "prior to," "before," or the like and as long as the output from a previous process is not used in a later process. Even if the process flow is described using phrases such as "first" or "next" in the claims, embodiments, or diagrams, it does not necessarily mean that the process must be performed in this order.

### [Explanation of Reference Symbols]

100: terminal; 110: acquiring unit; 112: camera; 114: optical element; 116: a pulse wave acquiring unit; 117: age estimating unit; 120: calculating unit; 122: low-pass filter; 123: high-pass filter; 124: frequency analyzing unit; 130: transmitting unit; 140: display unit; 200: processing apparatus; 210: receiving unit; 220: storage unit; 230: analyzing unit; 240: control unit; 250: reproducing apparatus; 260: output unit; 270: information determining unit; 300: bioinformation system; 310: measuring unit; 311: accelerometer; 312: blood pressure measuring unit

## Claims

1. An apparatus that stores information on a pulse wave of a living body, the apparatus comprising:
a receiving unit that receives a bias signal and a fundamental wave signal of the pulse wave, respectively; and
a storage unit that stores the bias signal and fundamental wave signal.

2. The apparatus according to claim 1, wherein the storage unit stores a higher harmonic wave signal of the pulse wave.

3. The apparatus according to claim 2, wherein the storage unit stores a second higher harmonic wave signal and a third higher harmonic wave signal of the pulse wave, respectively.

4. The apparatus according to claim 2 or 3, wherein the storage unit stores a frequency of the fundamental wave signal and an amplitude and a phase of the higher harmonic wave signal.

5. The apparatus according to any one of claims 1 to 4, wherein the storage unit stores reliability of the pulse wave.

6. The apparatus according to any one of claims 1 to 5, wherein the storage unit stores information on a pulse wave acquired from optical information of the living body.

7. The apparatus according to claim 6, wherein the storage unit stores information on a pulse wave acquired from video information of the living body.

8. The apparatus according to claim 6, wherein the storage unit stores information on pulse waves of two different colors acquired from optical information of the living body, respectively.

9. The apparatus according to any one of claims 1 to 8, wherein the storage unit stores information based on a shape of a face acquired from a facial image of the living body in association with information on the pulse wave.

10. The apparatus according to any one of claims 1 to 9, wherein the storage unit stores blood pressure information of the living body in association with information on the pulse wave.

11. The apparatus according to any one of claims 1 to 10, wherein a time interval of the bias signal stored by the storage unit is 0.5 to 1.3 seconds.

12. The apparatus according to any one of claims 1 to 11, wherein a time interval of the fundamental wave signal stored by the storage unit is 0.5 to 1.3 seconds.

13. The apparatus according to any one of claims 1 to 12, further comprising an analyzing unit that analyzes a state of the living body based on information on the pulse wave stored in the storage unit.

14. The apparatus according to claim 13, wherein the analyzing unit analyzes a state of the living body based on information on pulse waves of a plurality of living bodies.

15. The apparatus according to any one of claims 1 to 14, further comprising a reproducing apparatus that reproduces the pulse wave based on information on the pulse wave stored in the storage unit.
